# FASCICULE DE BREVET EUROPEEN

(11) **EP 2 616 180 B1**
(45) Date de publication et mention de la délivrance du brevet: **03.04.2019**
(21) Numéro de dépôt: 11773070.5
(22) Date de dépôt: 13.09.2011
(51) Int. Cl.: B03B 9/06, C05F 9/02, C05F 17/00, B02C 17/00, B09B 3/00, C12M 1/00

(54) **INSTALLATION PERFECTIONNÉE DE TRAITEMENT DE DÉCHETS**
VERBESSERTE ANLAGE ZUR MÜLLBEHANDLUNG
IMPROVED INSTALLATION FOR THE TREATMENT OF WASTE

(30) Priorité: 13.09.2010 FR 1057245
(43) Date de publication de la demande: 24.07.2013
(73) Titulaire: Alfyma Industrie, 77700 Bailly-Romainvilliers (FR)
(72) Inventeur: RAFFIN, Claude, 38920 CROLLES Cédex 275 (FR); VERDURAND, Yves, 38240 Meylan (FR); GIRARD-BLANC, Alain, 38400 Saint Martin D'heres (FR); WAROUX, Hervé, 38570 Le Cheylas (FR)
(74) Mandataire: Lavoix
(86) Numéro de dépôt international: PCT/FR2011/052085
(87) Numéro de publication internationale: WO 2012/035249

(56) Documents cités:
- EP-A2- 0 302 551
- CH-A5- 587 680
- GB-A- 921 007
- US-A- 3 930 799
- US-A- 4 203 376

## Description

La présente invention concerne une installation de traitement de déchets.

On connaît déjà, dans l'état de la technique, une installation de traitement de déchets, notamment de déchets ménagers, du type comportant un dispositif de fermentation des déchets. Les déchets ménagers sont introduits dans ce dispositif de fermentation, également appelé « tube de fermentation » ou « BRS » (acronyme pour « Bio Réacteurs Stabilisateurs »), pour y être dégradés par des bactéries. En effet, l'environnement dans le dispositif de fermentation, en particulier la température, la pression et l'humidité, est favorable à de telles bactéries.

Les déchets traités de cette manière sont généralement destinés à être convertis en compost.

Des installations de traitement de déchets sont par exemple décrites dans US 4 203 376, CH 587 680, EP 0302 551, US 3 930 799 ou GB 921 007.

Afin d'assurer une dégradation suffisante des déchets, ces déchets restent en moyenne trois jours dans le dispositif de fermentation, durant lesquels le dispositif de fermentation est accaparé. Ainsi, le cycle de traitement des déchets est généralement relativement long.

L'invention a notamment pour but d'améliorer le rendement d'une telle installation de traitement de déchets.

A cet effet, l'invention a notamment pour objet une installation de traitement de déchets selon la revendication 1.

Certains types de déchets, notamment les déchets fibreux tels que le papier ou le carton, doivent demeurer plus longtemps que d'autres dans le dispositif de fermentation afin d'y être dégradés de manière satisfaisante. L'invention propose d'effectuer un traitement préalable de ces types de déchets, en les faisant passer dans un dispositif de pré-déchiquetage avant de les introduire dans le dispositif de fermentation.

Le dispositif de pré-déchiquetage permet de déchiqueter ces déchets en petits morceaux, ou dans un cas optimal de convertir ces déchets en une substance boueuse, de façon à augmenter la surface spécifique de ces déchets, destinée à être attaquée par les bactéries du dispositif de fermentation. Cette réduction granulométrique permet d'améliorer la dégradation de ces déchets.

Ainsi, il apparaît que pour un traitement préalable de ces déchets d'environ 30 minutes dans le dispositif de pré-déchiquetage, la durée de fermentation nécessaire dans le dispositif de fermentation peut être réduite à un jour et demi, voire beaucoup moins dans certains cas favorables.

Ce traitement préalable d'une partie des déchets permet donc au moins de multiplier par deux le débit de traitement des déchets.

De préférence, une installation selon l'invention peut comporter l'une ou plusieurs des caractéristiques suivantes, prises seules ou selon toutes les combinaisons techniquement possibles.
- Le dispositif de pré-déchiquetage est propre à déchiqueter des déchets fibreux, tels que des papiers ou des cartons.
- Le dispositif de pré-déchiquetage comporte une enceinte de forme générale de révolution autour d'un axe, actionnable en rotation autour de cet axe, l'enceinte comportant au moins un compartiment de réception des déchets, délimité par une paroi munie de lames, notamment de lames dentées.
- Le dispositif de pré-déchiquetage comporte au moins deux compartiments, dont les parois sont délimitées l'une par rapport à l'autre par un épaulement.
- Le dispositif de pré-déchiquetage comporte des barres de relevage destinées à brasser les déchets dans l'enceinte en les entraînant d'un compartiment vers l'autre.
- Le dispositif de pré-déchiquetage comporte des pales inclinées disposées en sortie de l'enceinte, destinées à entraîner les déchets depuis l'enceinte vers la section du circuit de convoyage.
- Le dispositif de pré-déchiquetage est muni d'une voie d'arrivée de liquide, destinée à injecter du liquide dans l'enceinte, ou destinée à imbiber les déchets d'eau avant leur entrée dans l'enceinte, de façon à humidifier et imprégner ces déchets.
- Le circuit de convoyage comporte un embranchement muni de moyens de tri des déchets, destinés à séparer les déchets fibreux des autres déchets, de sorte que les déchets fibreux sont entraînés vers le dispositif de pré-déchiquetage par une première branche du circuit de convoyage, et les autres déchets sont entraînés vers le dispositif de fermentation par une seconde branche du circuit de convoyage.
- Les moyens de tri des déchets comportent des moyens de tri par granulométrie et/ou des moyens de tri balistique.

L'invention concerne également un procédé de traitement de déchets, selon la revendication 8.

L'invention sera mieux comprise à la lecture de la description qui va suivre, donnée uniquement à titre d'exemple et faite en se référant aux figures annexées parmi lesquelles :
- la Figure 1 représente schématiquement une installation selon un exemple de mode de réalisation de l'invention ;
- la Figure 2 est une vue en perspective d'un dispositif de pré-déchiquetage équipant l'installation de la Figure 1.

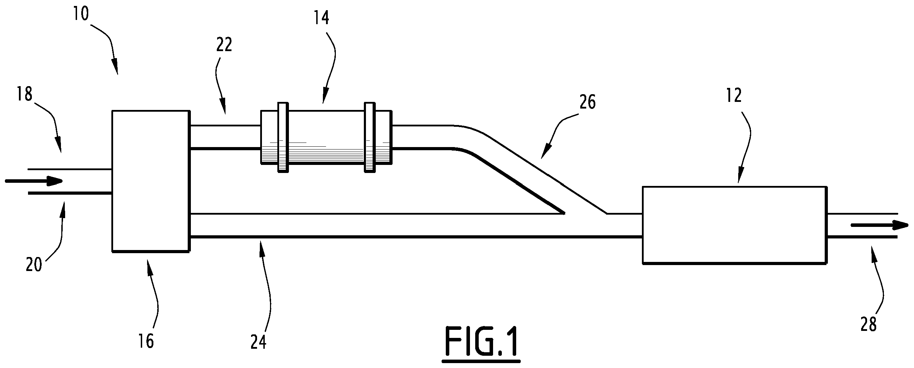

On a représenté sur la Figure 1 une installation 10 de traitement de déchets ménagers, du type comportant un dispositif 12 de fermentation des déchets.

Les déchets sont destinés à demeurer dans le dispositif de fermentation 12 pendant une durée de fermentation, durant laquelle les déchets sont dégradés de manière classique par action biologique de bactéries. Un tel dispositif de fermentation 12 présente généralement une enceinte tubulaire d'environ 4m de diamètre et d'environ 48m de longueur, dans laquelle demeurent les déchets.

Par ailleurs, l'installation 10 comporte un dispositif 14 de pré-déchiquetage d'au moins une partie des déchets, notamment celle majoritairement riche en papier carton.

Ce dispositif de pré-déchiquetage 14 est destiné à effectuer un traitement préalable d'une partie des déchets avant introduction dans le dispositif de fermentation 12, notamment par humidification et action mécanique.

De préférence, le dispositif de pré-déchiquetage 14 est un défibreur, propre à déchiqueter des déchets fibreux, tels que des papiers ou des cartons. En effet, ce sont généralement les déchets fibreux qui nécessitent les durées de dégradation les plus longues dans le dispositif de fermentation 12, dans le cas où ces déchets fibreux ne passent pas préalablement dans le dispositif de pré-déchiquetage 14 pour y être humidifiés, mouillés et/ou déchiquetés.

En effet, sans ce passage préalable, l'eau classiquement introduite dans le dispositif de fermentation 12 agit principalement sur les matières organiques (également appelées « fines ») qui, dans un deuxième temps seulement, permettent d'humidifier, d'imprégner et de désagréger les papiers cartons.

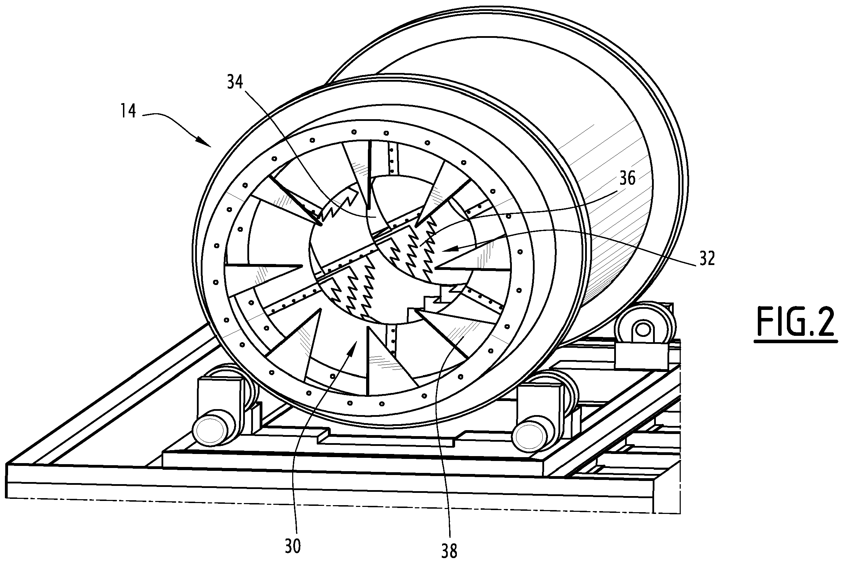

Le dispositif de pré-déchiquetage 14 sera décrit ultérieurement plus en détails en référence à la Figure 2.

Afin de trier préalablement les déchets fibreux parmi les autres déchets, l'installation de traitement 10 comporte des moyens 16 de tri des déchets, destinés à séparer majoritairement les déchets fibreux des autres déchets.

Ces moyens de tri des déchets comportent par exemple des moyens de tri par granulométrie de type classique. En effet, les déchets présentant une granulométrie comprise entre 100 et 300 mm sont généralement des déchets fibreux, tels que du papier ou du carton.

Les moyens de tri peuvent également comporter des moyens de tri balistique de type classique, seuls ou en combinaison avec les moyens de tri par granulométrie, afin d'affiner le tri.

L'installation de traitement 10 comporte enfin un circuit de convoyage 18 des déchets.

Le circuit de convoyage 18 comporte une section d'entrée 20, à laquelle sont déposés les déchets. Cette section d'entrée 20 débouche sur les moyens de tri 16.

Le circuit de convoyage 18 comporte également un embranchement, muni des moyens de tri 16, présentant une première branche 22 destinée à entraîner les déchets fibreux, préparés suite au tri, vers le dispositif de pré-déchiquetage 14, et une seconde branche 24 destinée à entraîner les autres déchets vers le dispositif de fermentation 12, notamment les déchets organiques.

Le circuit de convoyage 18 comporte en outre une section 26, reliant la sortie du dispositif de pré-déchiquetage 14 à l'entrée du dispositif de fermentation 12, pour l'introduction des déchets fibreux dans le dispositif de fermentation 12 après humidification et déchiquetage.

Le circuit de convoyage comporte enfin une section de sortie 28 par laquelle les déchets traités sont extraits après traitement dans les dispositifs de fermentation 12.

On a représenté sur la Figure 2 le dispositif de pré-déchiquetage 14.

Ce dispositif de pré-déchiquetage 14 comporte une enceinte 30 de forme générale de révolution autour d'un axe, actionnable en rotation autour de cet axe.

L'enceinte 30 présente par exemple un diamètre intérieur de 3 mètres et une longueur de 9 mètres. De préférence, cette enceinte 30 est destinée à entrer en rotation à une vitesse de 10 à 18 tours par minute.

L'enceinte 30 comporte au moins un compartiment de réception des déchets 32, de préférence trois compartiments adjacents. Chacun de ces compartiments est délimités par une paroi circulaire 34 munie de lames 36, notamment de lames dentées. Ces lames dentées 36 permettent de déchiqueter les déchets fibreux lorsqu'ils sont brassés par la rotation de l'enceinte 30.

Les compartiments 32 sont délimités les uns par rapport aux autres par des épaulements délimitant leurs parois 34.

Le dispositif de pré-déchiquetage 14 comporte également des barres de relevage destinées à brasser les déchets dans l'enceinte 30 en les entraînant progressivement d'un compartiment 32 à l'autre, en direction de la sortie du dispositif de pré-déchiquetage 14. Ces barres de relevage sont par exemple fixées aux parois 34 de manière oblique par rapport à l'axe de l'enceinte 30.

Avantageusement, le dispositif de pré-déchiquetage 14 est muni d'une voie d'arrivée de liquide, par exemple d'eau ou de boue, destinée à injecter ce liquide dans l'enceinte 30. En variante, la voie d'arrivée de liquide est dirigée vers la branche 22 d'arrivée des déchets fibreux, afin d'imbiber de liquide ces déchets fibreux avant leur entrée dans l'enceinte 30.

Ce liquide permet d'affaiblir les déchets fibreux et de favoriser leur déchiquetage par les lames dentées 36. De préférence, le liquide injectée dans l'enceinte 30 correspond à 20% de la masse entrante dans cette enceinte 30.

Du fait de ce traitement préalable, les déchets fibreux sont déchiquetés en petits morceaux ou, dans un cas optimal, en boue de déchets fibreux. Ainsi, ces déchets fibreux déchiquetés présentent une surface spécifique plus importante qu'avant le déchiquetage.

Le dispositif de pré-déchiquetage 14 comporte enfin des pales inclinées 38 disposées en sortie de l'enceinte 30, destinées à entraîner les déchets déchiquetés depuis l'enceinte 30 vers l'extérieur de cette enceinte 30, vers la section 26.

On notera que l'invention permet de réaliser un procédé de traitement de déchets, comportant une étape de pré-déchiquetage d'au moins une partie des déchets, notamment des déchets fibreux, dans le dispositif de pré-déchiquetage 14, une étape postérieure de convoyage des déchets déchiquetés vers le dispositif de fermentation 12, et une étape de fermentation des déchets dans le dispositif de fermentation 12.

Puisque les déchets fibreux, après déchiquetage, présentent une surface spécifique plus grande destinée à être attaquée par des bactéries, leur fermentation dans le dispositif de fermentation 12 est plus rapide. Ainsi, la durée de fermentation peut être réduite d'au moins un jour et demi.

L'invention n'est pas limitée au mode de réalisation précédemment décrit, mais pourrait présenter diverses variantes sans sortir du cadre de l'invention, tel que défini par les revendications ci-jointes.

## Revendications

1. Installation (10) de traitement de déchets, du type comportant un dispositif (12) de fermentation des déchets, **caractérisé en ce qu'**elle comporte :
- un dispositif (14) de pré-déchiquetage d'au moins une partie des déchets, propre à déchiqueter des déchets fibreux, tels que des papiers ou des cartons, et
- un circuit (18) de convoyage comportant une section (26) reliant la sortie du dispositif de pré-déchiquetage (14) à l'entrée du dispositif de fermentation (12),
et **en ce que** le circuit de convoyage (18) comporte un embranchement muni de moyens (16) de tri des déchets, destinés à séparer les déchets fibreux des autres déchets, de sorte que les déchets fibreux sont entraînés vers le dispositif de pré-déchiquetage (14) par une première branche (22) du circuit de convoyage (18), et les autres déchets sont entraînés vers le dispositif de fermentation (12) par une seconde branche (24) du circuit de convoyage (18).

2. Installation (10) selon la revendication 1, dans laquelle le dispositif de pré-déchiquetage (14) comporte une enceinte (30) de forme générale de révolution autour d'un axe, actionnable en rotation autour de cet axe, l'enceinte (30) comportant au moins un compartiment (32) de réception des déchets, délimité par une paroi (34) munie de lames (36), notamment de lames dentées.

3. Installation (10) selon la revendication 2, dans laquelle le dispositif de pré-déchiquetage (14) comporte au moins deux compartiments (32), dont les parois (34) sont délimitées l'une par rapport à l'autre par un épaulement.

4. Installation (10) selon la revendication 3, dans laquelle le dispositif de pré-déchiquetage (14) comporte des barres de relevage destinées à brasser les déchets dans l'enceinte en les entraînant d'un compartiment (32) vers l'autre.

5. Installation (10) selon l'une quelconque des revendications 2 à 4, dans laquelle le dispositif de pré-déchiquetage (14) comporte des pales inclinées (38) disposées en sortie de l'enceinte (30), destinées à entraîner les déchets depuis l'enceinte (30) vers la section (26) du circuit de convoyage (18).

6. Installation (10) selon l'une quelconque des revendications 2 à 5, dans laquelle le dispositif de pré-déchiquetage (14) est muni d'une voie d'arrivée de liquide, destinée à injecter du liquide dans l'enceinte, ou destinée à imbiber les déchets d'eau avant leur entrée dans l'enceinte (30), de façon à humidifier et imprégner ces déchets.

7. Installation (10) selon l'une quelconque des revendications précédentes, dans laquelle les moyens (16) de tri des déchets comportent des moyens de tri par granulométrie et/ou des moyens de tri balistique.

8. Procédé de traitement de déchets, exécuté dans une installation selon l'une quelconque des revendications précédentes.

## Patentansprüche

1. Einrichtung (10) zur Abfallentsorgung, umfassend eine Einrichtung (12) zur Fermentation der Abfälle, **dadurch gekennzeichnet, dass** sie umfasst:
- eine Vorrichtung (14) zur Vorzerkleinerung mindestens eines Teils der Abfälle, die dazu geeignet ist, die faserigen Abfälle wie z.B. Papier oder Pappe zu zerkleinern, und
- einen Förderkreislauf (18), der einen Abschnitt (26), der den Ausgang der Vorzerkleinerungsvorrichtung (14) mit dem Eingang der Fermentationseinrichtung (12) verbindet,
und dass der Förderkreislauf (18) eine Abzweigung mit Mitteln (16) zur Abfalltrennung umfasst, um die faserigen Abfälle von den anderen Abfällen zu trennen, sodass die faserigen Abfälle durch einen ersten Zweig (22) des Förderkreislaufs (18) der Vorzerkleinerungsvorrichtung (14) zugeführt werden und die anderen Abfälle durch einen zweiten Zweig (24) des Förderkreislaufs (18) der Fermentationseinrichtung (12) zugeführt werden.

2. Einrichtung (10) nach Anspruch 1, wobei die Vorzerkleinerungsvorrichtung (14) ein Gehaüse (30) dessen Form ein Rotationskörper um eine Achse ist und die um diese Achse rotierbar ist, wobei das Gehaüse(30) mindestens einen Bereich (32) zur Aufnahme von Abfällen, der durch eine Wand (34) mit Klingen (36), insbesondere gezahnten Klingen, abgegrenzt ist.

3. Einrichtung (10) nach Anspruch 2, wobei die Vorzerkleinerungseinrichtung (14) mindestens zwei Bereiche (32) umfasst, deren Wände (34) durch eine Schulter voneinander abgegrenzt sind.

4. Einrichtung (10) nach Anspruch 3, wobei die Vorzerkleinerungsvorrichtung (14) Hubstangen umfasst, um die Abfälle in dem Gehaüse zu rühren und sie von einem Bereich (32) zu einem anderen zu bewegen.

5. Einrichtung (10) nach einem der Ansprüche 2 - 4, wobei die Vorzerkleinerungsvorrichtung (14) geneigte Schaufelblätter (38) umfasst, die am Ausgang des Gehaüses (30) angeordnet sind, um die Abfälle von dem Gehaüse (30) zum Abschnitt (26) des Förderkreislaufs (18) zu bringen.

6. Einrichtung (10) nach einem der Ansprüche 2 - 5, wobei die Vorzerkleinerungseinrichtung (14) mit einem Flüssigkeitszulauf zum Einspritzen einer Flüssigkeit in das Gehaüse oder zum Tränken der Abfälle mit Wasser vor ihrem Eintritt in das Gehaüse (30) ausgestattet ist, um die Abfälle zu befeuchten und imprägnieren.

7. Einrichtung (10) nach einem der vorstehenden Ansprüche, wobei die Mittel (16) zum Trennen der Abfälle Mittel zur granulometrischen und/oder ballistischen Trennung umfassen.

8. Verfahren zur Entsorgung von Abfällen, das in einer Einrichtung nach einem der vorstehenden Ansprüche ausgeführt wird.

## Claims

1. A waste treatment facility (10), of the type comprising a device (12) for fermentation of the waste, **characterized in that** it comprises:
- a device (14) for pre-shredding of at least part of the waste, capable of shredding fibrous waste, such as paper or cardboard, and
- a conveying circuit (18) comprising a section (26) connecting the outlet of the pre-shredding device (14) to the inlet of the fermentation device (12),
and **in that** the conveying circuit (18) comprises a spur provided with waste sorting means (16), intended to separate the fibrous waste from the other waste, such that the fibrous waste is driven toward the pre-shredding device (14) by a first branch (22) of the conveying circuit (18), and the other waste is driven toward the fermentation device (12) by a second branch (24) of the conveying circuit (18).

2. The facility (10) according to claim 1, wherein the pre-shredding device (14) comprises a chamber (30) with a general shape of revolution around an axis, able to be actuated in rotation around said axis, the chamber (30) comprising at least one compartment (32) for receiving waste, delimited by a wall (34) provided with blades (36), in particular toothed blades.

3. The facility (10) according to claim 2, wherein the pre-shredding device (14) comprises at least two compartments (32), the walls (34) of which are delimited relative to one another by a shoulder.

4. The facility (10) according to claim 3, wherein the pre-shredding device (14) comprises lifting bars intended to mix the waste in the chamber by driving it from one compartment (32) toward the other.

5. The facility (10) according to any one of claims 2 to 4, wherein the pre-shredding device (14) comprises inclined blades (38) arranged at the outlet of the chamber (30), intended to drive the waste from the chamber (30) toward the section (26) of the conveying circuit (18).

6. The facility (10) according to any one of claims 2 to 5, wherein the pre-shredding device (14) is provided with a liquid intake channel, intended to inject liquid into the chamber, or intended to soak the waste with water before it enters the chamber (30), so as to moisten and impregnate said waste.

7. The facility (10) according to any one of the preceding claims, wherein the waste sorting means (16) comprise means for sorting by particle size and/or ballistic sorting means.

8. A waste treatment method, carried out in a facility according to any one of the preceding claims.
